# EUROPÄISCHE PATENTANMELDUNG

(11) **EP 4 059 525 A1**
(43) Veröffentlichungstag der Anmeldung: **21.09.2022**
(21) Anmeldenummer: 22161505.7
(22) Anmeldetag: 11.03.2022
(51) Int. Cl.: A61L 2/00, A61L 2/18, A47K 5/12, A61B 42/40

(54) **EINSATZAUSRÜSTUNG**

(30) Priorität: 15.03.2021 DE 102021106183
(71) Anmelder: Bonowi International Police Equipment GmbH, 55129 Mainz-Hechtsheim (DE)
(72) Erfinder: CORUMLU, Cihan, 36043 Fulda (DE)
(74) Vertreter: Wolf & Wolf Patent- und Rechtanwaltsgesellschaft mbH

(57) **Zusammenfassung**

Die Erfindung betrifft eine Einsatzausrüstung, umfassend ein Behältnis (1) und einen im Behältnis (1) angeordneten Desinfektionsmittelbehälter (2), wobei der Desinfektionsmittelbehälter (2) mit einem von außerhalb des Behältnisses (1) zugänglichen Desinfektionsmittelspender (3) verbunden ausgebildet ist. Nach der Erfindung ist vorgesehen, dass im Behältnis (1) neben dem Desinfektionsmittelbehälter (2) ein ebenfalls von außerhalb des Behältnisses (1) zugängliches Handschuhfach (4) angeordnet ist.

## Beschreibung

Die Erfindung betrifft eine Einsatzausrüstung gemäß dem Oberbegriff des Patentanspruchs 1.

Eine Einsatzausrüstung der eingangs genannten Art ist beispielsweise aus dem Dokument US 2007/0051752 A1 bekannt. Diese Einsatzausrüstung, die wie die noch zu erläuternde, erfindungsgemäße Einsatzausrüstung insbesondere für den Polizeieinsatz gedacht und somit tragbar bzw. zum Tragen ausgebildet ist, besteht aus einem Behältnis und einem im Behältnis (also in einem Innenraum des Behältnisses bzw. des Behälters) angeordneten Desinfektionsmittelbehälter, wobei der Desinfektionsmittelbehälter mit einem von außerhalb des Behältnisses zugänglichen Desinfektionsmittelspender verbunden ausgebildet ist. Bei dieser Lösung ist das besagte Behältnis dabei (soweit ersichtlich) taschenartig und aus einem weichen, leicht knickbaren Material gebildet.

Der Erfindung liegt die Aufgabe zugrunde eine Einsatzausrüstung zu verbessern. Insbesondere soll eine den heutzutage realen Einsatzbedingungen noch gerechter werdende Einsatzausrüstung geschaffen werden.

Diese Aufgabe ist mit einer Einsatzausrüstung der eingangs genannten Art durch die im Kennzeichen des Patentanspruchs 1 aufgeführten Merkmale gelöst.

Nach der Erfindung ist also vorgesehen, dass im Behältnis neben dem Desinfektionsmittelbehälter ein ebenfalls von außerhalb des Behältnisses zugängliches Handschuhfach angeordnet ist.

Mit anderen Worten zeichnet sich die erfindungsgemäße Lösung somit dadurch aus, dass die Einsatzausrüstung nunmehr eine Doppelfunktion aufweist, nämlich einerseits die Bereitstellung einer Desinfektionsfunktion und andererseits die Bereitstellung von Handschuhen, also einer weiteren Ausrüstung, die die Umsetzung eines möglichen Hygienekonzeptes erheblich erleichtert.

Andere vorteilhafte Weiterbildungen der erfindungsgemäßen Einsatzausrüstung ergeben sich aus den abhängigen Patentansprüchen.

Der Vollständigkeit halber wird noch auf folgende Dokumente hingewiesen: EP 1 906 859 B1, US 6,021,920 A, US 4,280,643 A und DE 20 2018 001 468 U1.

Die erfindungsgemäße Einsatzausrüstung einschließlich ihrer vorteilhaften Weiterbildungen gemäß der abhängigen Patentansprüche wird nachfolgend anhand der zeichnerischen Darstellung verschiedener Ausführungsbeispiele näher erläutert.

Es zeigt perspektivisch
- Figur 1: in Vorderansicht die erfindungsgemäße Einsatzausrüstung;
- Figur 2: in Rückansicht eine Ausführungsform mit einer Befestigungsklettfläche; und
- Figur 3: in Rückansicht eine weitere Ausführungsform mit zwei Befestigungshaken.

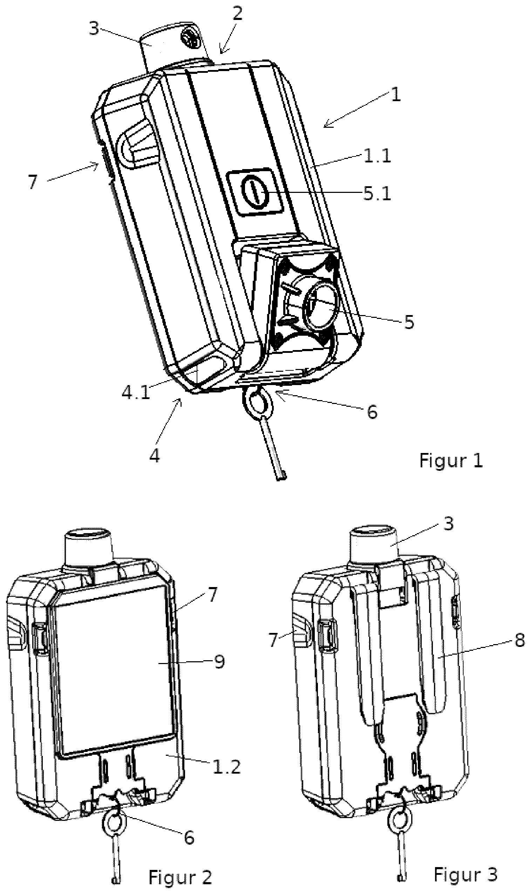

Die in den Figuren 1 bis 3 dargestellte Einsatzausrüstung besteht zunächst in bekannter Weise aus einem Behältnis 1 und einem im Behältnis 1 angeordneten Desinfektionsmittelbehälter 2, wobei der Desinfektionsmittelbehälter 2 mit einem von außerhalb des Behältnisses 1 zugänglichen Desinfektionsmittelspender 3 verbunden ausgebildet ist.

Wesentlich für die erfindungsgemäße Einsatzausrüstung ist nun, dass im Behältnis 1 neben dem Desinfektionsmittelbehälter 2 ein ebenfalls von außerhalb des Behältnisses 1 zugängliches Handschuhfach 4 angeordnet ist. Das Behältnis 1 weist dabei - abgesehen vom Handschuhfach 4 - vorzugsweise einen im Einsatz von außen unzugänglichen Innenraum auf.

Um möglichst leicht an die im Handschuhfach 4 gelagerten Handschuhe gelangen zu können, ist mit Verweis auf Figur 1 am Behältnis 1 bevorzugt eine zum Handschuhfach 4 führende Öffnung 4.1 vorgesehen. Diese Öffnung 4.1 ist dabei besonders bevorzugt verschlussfrei ausgebildet, d. h. sie ist einfach offen. Dabei ist weiterhin bevorzugt vorgesehen, dass die Öffnung 4.1 eine ein Herausfallen von im Handschuhfach 4 gelagerten Handschuhen, vorzugsweise Einweghandschuhen, verhindernde Größe aufweist. Das Handschuhfach 4 selbst ist dabei so groß ausgebildet, dass es mindestens zwei Paar Handschuhe aufnehmen kann.

Wie weiterhin aus den Figuren ersichtlich, ist außerdem bevorzugt vorgesehen, das Behältnis 1 angenähert quaderförmig ausgebildet ist. Ferner ist bevorzugt vorgesehen, dass das Behältnis 1 aus Kunststoff gebildet ist. Insbesondere im Unterschied zum eingangs genannten Stand der Technik ist besonders bevorzugt vorgesehen, dass das Behältnis 1 formstabil ausgebildet ist, d. h. es besteht insbesondere zum Beispiel nicht aus Gewebe oder dergleichen, sondern aus einem steifen, wenn überhaupt nur geringfügig verformbaren Material. Ferner ist bevorzugt vorgesehen, dass das Behältnis 1 vollständig in einen gedachten Würfel mit 20 cm, vorzugsweise 15 cm, Seitenlänge passt.

Mit Verweis auf die Ausführungsform gemäß Figur 2 ist weiterhin besonders bevorzugt vorgesehen, dass das Behältnis 1 mindestens eine Befestigungsklettfläche 9 aufweist. Mit dieser kann die Einsatzausrüstung auf besonders einfache Weise zum Beispiel an einem Oberbekleidungsstück (mit Gegenklettfläche) einer Einsatzperson befestigt werden.

Alternativ ist mit Verweis auf Figur 3 bevorzugt vorgesehen, dass das Behältnis 1 mindestens einen Befestigungshaken 8 aufweist (Figur 3 zeigt zwei Befestigungshakten 8). Mit diesem kann die Einsatzausrüstung auf besonders einfache Weise zum Beispiel an einem Gürtel einer Einsatzperson befestigt werden.

In beiden Fällen ist dabei bevorzugt vorgesehen, dass das quaderförmige Behältnis 1 eine Rückseite 1.2 zur Anordnung wahlweise des Befestigungshakens 8 und/oder der Befestigungsklettfläche 9 aufweist.

Bezüglich des vorgenannten Desinfektionsmittelspenders 3 ist bevorzugt vorgesehen, dass dieser als Sprühkopf ausgebildet ist. Außerdem ist zur Gewährleistung einer guten Zugänglichkeit bevorzugt vorgesehen, dass der Desinfektionsmittelspender 3 an einem bei bestimmungsgemäßen Gebrauch oberen Ende des Behältnisses 1 angeordnet ist.

Weiterhin ist besonders bevorzugt vorgesehen, dass das Behältnis 1 zum Austausch des Desinfektionsmittelbehälters 2 sich öffnen und schließen lassend ausgebildet ist. Hierzu ist ferner besonders bevorzugt vorgesehen, dass das Behältnis 1 zum Öffnen und Schließen mit einem vorzugsweise werkzeugfrei zu betätigenden Schnappverschluss 7 versehen ausgebildet ist.

Darüber hinaus ist wiederum mit Verweis auf Figur 1 besonders bevorzugt vorgesehen, dass das Behältnis 1 mit mindestens einer Lampe 5 versehen ausgebildet ist. Dabei ist bevorzugt vorgesehen, dass das quaderförmige Behältnis 1 eine Vorderseite 1.1 zur Anordnung der Lampe 5 aufweist. Weiterhin ist bevorzugt vorgesehen, dass die bevorzugt batteriebetriebene Lampe 5 verstellbar am Behältnis 1 befestigt ist. Noch etwas genauer betrachtet, ist dabei bevorzugt vorgesehen, dass die Lampe 5 um eine bei bestimmungsgemäßen Gebrauch des Behältnisses 1 horizontal verlaufende Achse schwenkbar ausgebildet ist.

Außerdem ist in diesem Zusammenhang bevorzugt am Behältnis 1 ein Schalter 5.1 zum Ein- und Ausschalten der Lampe 5 vorgesehen. Dieser Schalter 5.1 ist dabei, wie in Figur 1 dargestellt, besonders bevorzugt auf einer Vorderseite 1.1 des Behältnisses 1 angeordnet.

Schließlich ist besonders bevorzugt vorgesehen, dass der Behältnis 1 ein Schlüsselbefestigungselement 5 zum Beispiel für einen Schlüssel von Handschellen aufweist. Dabei ist besonders bevorzugt vorgesehen (allerdings in den Figuren nicht im Detail dargestellt), dass das Schlüsselbefestigungselement 6 als Teil eines ausziehbaren Schlüsselbandes ausgebildet ist.

Wie aus alledem ersichtlich, steht einer Einsatzperson, zum Beispiel von der Polizei, mit der erfindungsgemäßen Einsatzausrüstung eine universelle "Medibox" zur Verfügung, mit deren Hilfe sich ein Einsatz zum Vorteil aller Beteiligten besonders hygienisch gestalten lässt. Die erfindungsgemäße Einsatzausrüstung ist somit insbesondere zur Befestigung an einer Person ausgebildet.

### Bezugszeichenliste

- 1: Behältnis
- 1.1: Vorderseite
- 1.2: Rückseite
- 2: Desinfektionsmittelbehälter
- 3: Desinfektionsmittelspender
- 4: Handschuhfach
- 4.1: Öffnung
- 5: Lampe
- 5.1: Schalter
- 6: Schlüsselbefestigungselement
- 7: Schnappverschluss
- 8: Befestigungshaken
- 9: Befestigungsklettfläche

## Patentansprüche

1. Einsatzausrüstung, umfassend ein Behältnis (1) und einen im Behältnis (1) angeordneten Desinfektionsmittelbehälter (2), wobei der Desinfektionsmittelbehälter (2) mit einem von außerhalb des Behältnisses (1) zugänglichen Desinfektionsmittelspender (3) verbunden ausgebildet ist,
**dadurch gekennzeichnet,**
**dass** im Behältnis (1) neben dem Desinfektionsmittelbehälter (2) ein ebenfalls von außerhalb des Behältnisses (1) zugängliches Handschuhfach (4) angeordnet ist.

2. Einsatzausrüstung nach Anspruch 1,
**dadurch gekennzeichnet,**
**dass** am Behältnis (1) eine zum Handschuhfach (4) führende Öffnung (4.1) vorgesehen ist.

3. Einsatzausrüstung nach Anspruch 2,
**dadurch gekennzeichnet,**
**dass** die Öffnung (4.1) verschlussfrei ausgebildet ist.

4. Einsatzausrüstung nach Anspruch 2 oder 3,
**dadurch gekennzeichnet,**
**dass** die Öffnung (4.1) eine ein Herausfallen von im Handschuhfach (4) gelagerten Handschuhen verhindernde Größe aufweist.

5. Einsatzausrüstung nach einem der Ansprüche 1 bis 4,
**dadurch gekennzeichnet,**
**dass** der Desinfektionsmittelspender (3) als Sprühkopf ausgebildet ist.

6. Einsatzausrüstung nach einem der Ansprüche 1 bis 5,
**dadurch gekennzeichnet,**
**dass** der Desinfektionsmittelspender (3) an einem bei bestimmungsgemäßen Gebrauch oberen Ende des Behältnisses (1) angeordnet ist.

7. Einsatzausrüstung nach einem der Ansprüche 1 bis 6,
**dadurch gekennzeichnet,**
**dass** das Behältnis (1) zum Austausch des Desinfektionsmittelbehälters (2) sich öffnen und schließen lassend ausgebildet ist.

8. Einsatzausrüstung nach einem der Ansprüche 1 bis 7,
**dadurch gekennzeichnet,**
**dass** das Behältnis (1) formstabil ausgebildet ist.

9. Einsatzausrüstung nach einem der Ansprüche 1 bis 8,
**dadurch gekennzeichnet,**
**dass** das Behältnis (1) mit mindestens einer Lampe (5) versehen ausgebildet ist.

10. Einsatzausrüstung nach einem der Ansprüche 1 bis 9,
**dadurch gekennzeichnet,**
**dass** der Behältnis (1) ein Schlüsselbefestigungselement (5) aufweist.
